# EUROPEAN PATENT APPLICATION

(11) **EP 4 800 112 A1**
(43) Date of publication of application: **02.09.2026**
(21) Application number: 24882866.7
(22) Date of filing: 24.10.2024
(51) Int. Cl.: C12N 15/73, C12N 7/00, C12N 15/113, C12N 9/22

(54) **CRISPR-CAS-LOADED BACTERIOPHAGE AND USE THEREOF**

(30) Priority: 24.10.2023 KR 20230143307
(71) Applicant: Chung Ang University Industry Academic Cooperation Foundation, Seoul 06974 (KR)
(72) Inventor: LEE, Sang Jun, Anseong-si Gyeonggi-do 17546 (KR); LEE, Ho Joung, Anseong-si Gyeonggi-do 17546 (KR); JEONG, Song Hee, Anseong-si Gyeonggi-do 17546 (KR); LEE, Chan Kyeong, Anseong-si Gyeonggi-do 17546 (KR)
(74) Representative: dompatent
(86) International application number: PCT/KR2024/016322
(87) International publication number: WO 2025/089839

(57) **Abstract**

The present invention relates to an engineered phage loaded with CRISPR S-Cas and a use thereof. The CRISPR-Cas-loaded bacteriophage according to the present invention enables sequence-specific microbial control and effectively suppresses the emergence of lysogenic strains, and thus is expected to be widely used throughout biotechnology such as environmental, agricultural, medical, and industrial biotechnology.

## Description

### [Technical Field]

The present invention relates to a CRISPR-Cas-loaded bacteriophage and uses thereof, and more particularly, to a method of producing a phage loaded with a CRISPR-Cas system to specifically recognize and cleave a specific nucleotide sequence in the genome of an infected microbial host, thereby suppressing the emergence of lysogenic strains and completely killing microorganisms infected with the phage.

### [Background Art]

Bacteriophages infect host microorganisms, replicate and assemble their own genomes, and then lyse the host cells to release progeny phages into the external environment. Bacteriophage infection begins with specific attachment to the receptors of host microorganisms, resulting in high infection specificity, and generally, has little effect on humans, animals or plants as well as other species of microorganisms. Such high host specificity is advantageous in terms of safety, and thus bacteriophages are attracting attention as antimicrobial agents with high specificity, which can replace antibiotics.

Most bacteriophages have either a lytic or lysogenic life cycle. Among bacteriophages with a lysogenic life cycle, some may insert phage genomes into the host genomes in the form of a prophage. Such lysogenic phages may achieve superinfection exclusion, thereby preventing DNA invasion or suppressing the lytic life cycle during subsequent infections by similar phages. This characteristic acts as resistance to bacteriophages, increasing the possibility of the emergence of resistance to the bacteriophages when controlling microorganisms through repeated phage infection.

Researchers from McMaster University in Canada have developed a microbial control method that suppresses the emergence of lysogenic strains by using the synergistic effect of the combination of lysogenic bacteriophages with antibiotics (Al-Anany A.M. et al. 2021. Cell Rep. PMID 34038739). Researchers from Zhejiang University in China and the University of Edinburgh in Scotland induced selective killing of *Shigella flexneri* pathogens using a P1 phagemid carrying *cas9* gene (Huan Y.W. et al. 2023, ACS Synth. Biol. PMID: 36802585). Researchers from SNIPR BIOME in Denmark (Gencay Y.E. et al. 2023. Nat. Biotechnol. PMID: 37142704), and researchers from Henan University of Education in China (Jin M. et al. 2022, Microbiol. Spectr. PMID: 35876591) reported methods for selective killing of *E. coli* by constructing bacteriophages with integrated CRISPR cascade and CRISPR arrays.

The above reports and inventions respectively have the following limitations. The combination of lysogenic phages with antibiotics may affect non-target microorganisms, and there is no clear solution to overcome the emergence of additional resistance. Microbial control using the Cas9-P1 phagemid requires cloning the phagemid when changing a target microorganism and has the limitation that the P1 phage packaging system must be continuously borrowed and used during the formation of progeny phages. For the phages carrying CRISPR cascade and CRISPR array, the large size of the CRISPR gene construct to be inserted into the phages limits their application to bacteriophages with small genomes to allow additional insertion into phage genomes. Therefore, there is a need to develop a bacteriophage-based microbial control method, which can solve the problem of bacteriophage resistance arising from the emergence of lysogenic strains through a highly target-specific CRISPR-Cas delivery method that targets a DNA sequence in the microbial host genome and can be broadly applied to diverse phages.

### [Disclosure]

### [Technical Problem]

Under the circumstances described above, the present inventors sought to develop a bacteriophage loaded with a CRISPR-Cas system, which has sequence-specific killing ability against a target microorganism and does not generate a lysogenic strain. Accordingly, the present inventors completed the present invention by inserting a Cas gene and guide RNA (gRNA) recognizing a target into the genome of bacteriophage λ and identifying the effects of sequence-specific killing ability against a target microorganism and prevention of the emergence of lysogenic strains in plaques formed by killing.

Accordingly, an object of the present invention is to provide a method of killing a microbial strain, comprising treating a microbial strain with a bacteriophage having inserted into the genome a CRISPR-Cas system comprising a donor nucleic acid molecule and a gRNA, wherein the donor nucleic acid molecule and the gRNA complementarily bind to a target DNA, which is a gene specific to the microbial strain.

Another object of the present invention is to provide a method of constructing a lytic bacteriophage, comprising inserting into a bacteriophage genome a CRISPR-Cas system comprising a donor nucleic acid molecule and a gRNA, wherein the donor nucleic acid molecule and the gRNA complementarily bind to a target DNA, which is a gene specific to the microbial strain, and a lytic bacteriophage constructed according to the method of constructing the lytic bacteriophage.

Yet another object of the present invention is to provide a method of avoiding superinfection immunity of a lysogenic microbial strain, comprising treating a lysogenic microbial strain with the bacteriophage, and a composition for avoiding superinfection immunity of a lysogenic microbial strain, comprising the bacteriophage.

### [Technical Solution]

In order to achieve the above object, the present invention provides a method of killing a microbial strain, comprising treating a microbial strain with a bacteriophage having inserted into the genome a CRISPR-Cas system comprising a donor nucleic acid molecule and a gRNA, wherein the donor nucleic acid molecule and the gRNA complementarily bind to a target DNA, which is a gene specific to the microbial strain.

Additionally, the present invention provides a method of constructing a lytic bacteriophage, comprising inserting into a bacteriophage genome a CRISPR-Cas system comprising a donor nucleic acid molecule and a gRNA, wherein the donor nucleic acid molecule and the gRNA complementarily bind to a target DNA, which is a gene specific to the microbial strain, and a lytic bacteriophage constructed according to the method of constructing the lytic bacteriophage.

Additionally, the present invention provides a method of avoiding superinfection immunity of a lysogenic microbial strain, comprising treating a lysogenic microbial strain with the bacteriophage, and a composition for avoiding superinfection immunity of a lysogenic microbial strain, comprising the bacteriophage.

### [Advantageous Effects]

The present invention relates to a CRISPR-Cas-loaded bacteriophage and uses thereof, and the microbial control technology according to the present invention may be broadly utilized even in a bacteriophage platform having limited extra space in the genome by loading only the minimum genes required for microbial control, may prevent the emergence of lysogenic strains through control of specifically recognizing the nucleotide sequence of a virulence gene in the genome of a harmful pathogenic microorganism and cleaving DNA, and may allow microorganisms not having a virulence gene sequence to become lysogenic, thereby enabling maintenance of ecological balance through selective killing rather than indiscriminate microbial killing, and thus may be utilized in the future to selectively control only harmful pathogenic microorganisms in the human body and environment.

### [Description of Drawings]

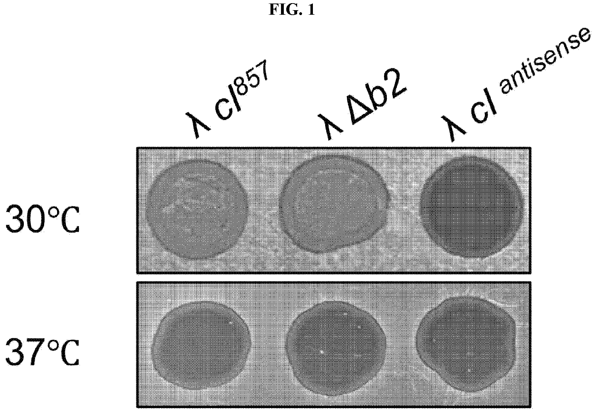

FIG. 1 shows spotting assay results of bacteriophages λ *cI*⁵⁸⁷, λ Δ*b2*, and λ *cI^{antisense}* on soft agar (30 °C and 37 °C) containing *Escherichia coli* MG1655 cells, wherein clear spots and turbid spots respectively indicate lytic cell death and lysogenic strain formation.

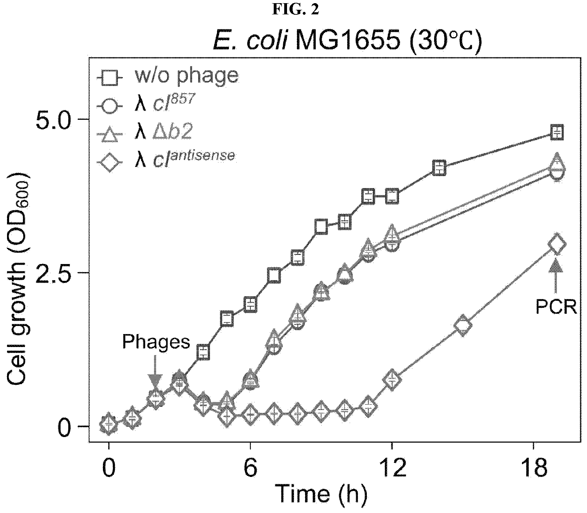

FIG. 2 shows a graph analyzing the growth of *E*. *coli* MG1655 cells infected with bacteriophages in liquid medium at 30 °C, wherein downward gray arrows indicate the time point of phage infection, and upward red arrows indicate the time point of sample collection for PCR. The growth measurement results represent average values obtained from three independent cultures.

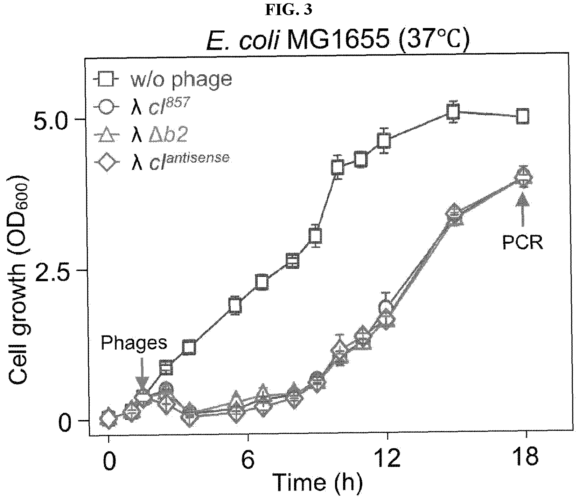

FIG. 3 shows a graph analyzing the growth of *E*. *coli* MG1655 cells infected with bacteriophages in liquid medium at 37 °C, wherein downward gray arrows indicate the time point of phage infection, and upward red arrows indicate the time point of sample collection for PCR. The growth measurement results represent average values obtained from three independent cultures.

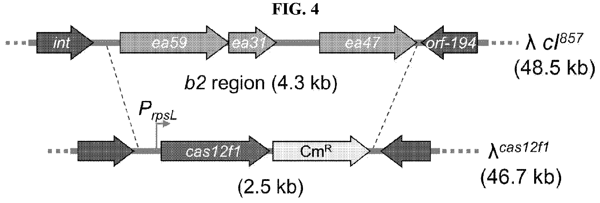

FIG. 4 shows the genomic structure of λ*^{cas12f1}*, wherein the *cas12f1* gene was inserted into the b2 region of the λ prophage genome through homologous recombination.

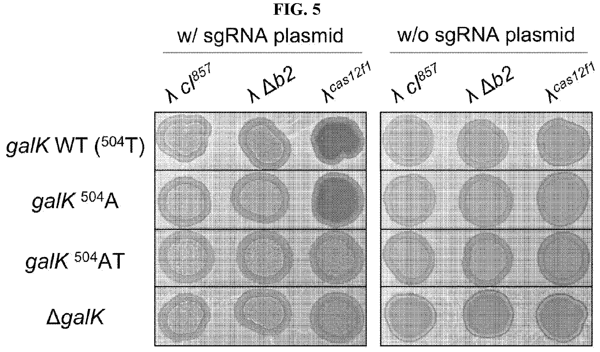

FIG. 5 shows spotting assay results of λ*^{cas12f1}*. *E*. *coli* MG1655 cells having various *galK* genotypes (*galK* WT, *galK* ⁵⁰⁴A, *galK* ⁵⁰⁴AT, and Δ*galK*) were transformed to include or not include a *galK*-targeting *sgRNA* plasmid, and λ *cI⁸⁵⁷*, λ Δ*b2*, and λ*^{cas12f1}* phages were spotted on soft agar containing the transformed cell culture, followed by incubation at 30 °C for 16 hours, and then a spotting assay was performed.

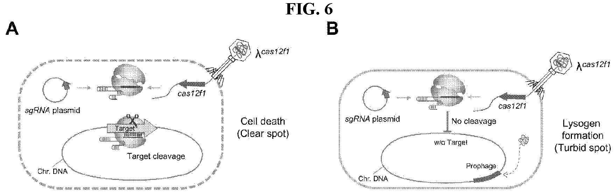

FIGS. 6A and 6B schematically illustrate the mechanism of cell killing by λ*^{cas12f1}*. The Cas12f1-sgRNA complex cleaves host genomic DNA when a target sequence is present, thereby causing cell death (A). The Cas12f1 nuclease is delivered by an external phage λ carrying the *cas12f1* gene, and the sgRNA is supplied by a plasmid in the host cell. In the absence of a target sequence, cleavage does not occur, so that the host cell survives and is potentially lysogenized (B). This may allow only target cells to be lysed while non-target cells survive.

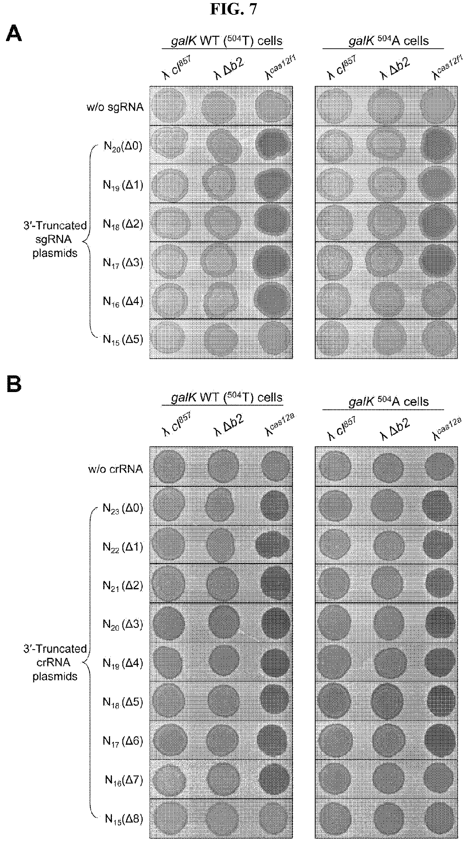

FIGS. 7A and 7B show spotting assay results of λ*^{cas12f1}* or λ*^{cas12a}*, respectively. λ *cI⁸⁵⁷*, λ Δ*b2*, λ*^{cas12f1}*, or λ*^{cas12a}* phages were spotted on soft agar containing MG1655 *galK* WT and ⁵⁰⁴A cell cultures carrying transformed truncated sgRNA or crRNA of different lengths, followed by incubation at 30 °C for 16 hours. Δ0-Δ5 and Δ0-Δ8 respectively indicate the number of 3'-terminal truncations of sgRNA of Cas12f1 and Cas12a, and N₂₀-N₁₅ and N₂₃-N₁₅ respectively indicate the length of the target recognition sequence (TRS) of sgRNA of Cas12f1 or crRNA of Cas12a.

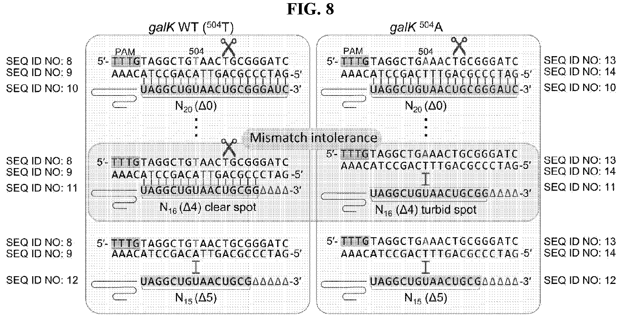

FIG. 8 schematically illustrates the mismatch intolerance mechanism of Cas12f1. The complex formed by 3'-terminal-truncated sgRNA and the Cas12f1 nuclease may distinguish a single nucleotide variation in a genomic target. 504 indicates the nucleotide position within the *galK* structural gene. The red nucleotide A indicates a variation at the 504th nucleotide position. The black vertical lines indicate base pairing between the non-PAM strand of the target DNA and the sgRNA. The scissors icon indicates that the Cas12f1-sgRNA complex recognizes and cleaves the target DNA. The Δ symbol indicates nucleotides truncated from the sgRNA. The red I symbol indicates that the target DNA sequence is not recognized by the sgRNA as a cleavage target.

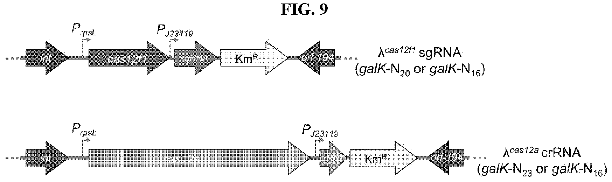

FIG. 9 respectively shows the structure of synthetic λ phages carrying both a *cas12f1* gene-*galK*-targeting *sgRNA* gene or a *cas12a* gene-*galK*-targeting *crRNA* gene. Phages having inserted into the λ *cI⁸⁵⁷* genome *cas12f1* or *cas12a* and a *galK*-targeting *sgRNA* or *crRNA* gene were constructed. λ*^{cas12f1}galK*-N₂₀ and λ*^{cas12f1}galK*-N₁₆ have sgRNA having target recognition sequences of 20 nt and 16 nt in length, respectively, and λ*^{cas12a}galK*-N₂₃ and λ*^{cas12a}galK*-N₁₆ have crRNA having target recognition sequences of 23 nt to 16 nt in length.

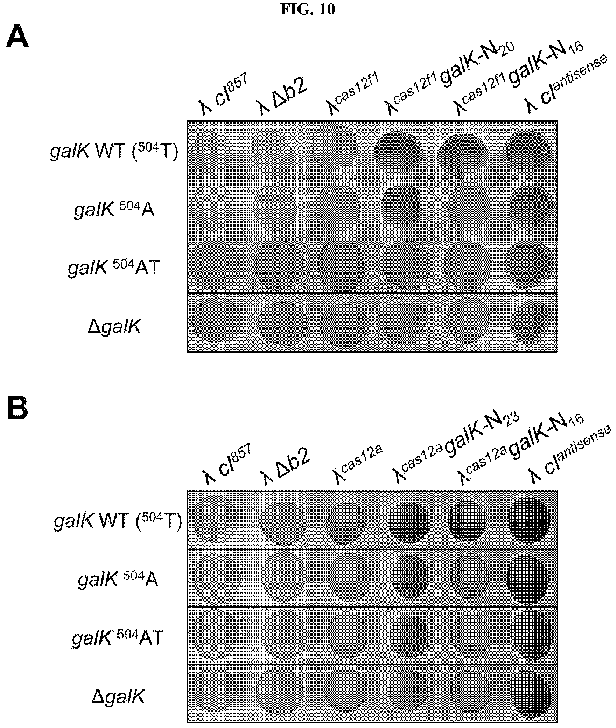

FIGS. 10A and 10B show spotting assay results of synthetic phages including a *cas12f1-sgRNA*-loaded phage (A) or a *cas12a-crRNA*-loaded phage (B), respectively, on soft agar containing MG1655, MG1655-*galK* ⁵⁰⁴A, MG1655-*galK* ⁵⁰⁴AT, and MG1655-Δ*galK* cells for 16 hours at 30 °C.

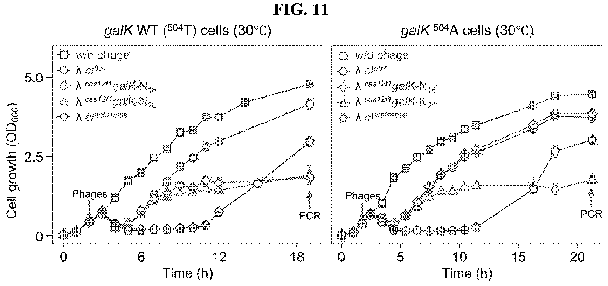

FIG. 11 shows results indicating genome *galK* sequence-specific bacterial control by a *cas12f1-sgRNA*-loaded phage. At 30 °C, the growth of MG1655 and MG1655-*galK* ⁵⁰⁴A strains was measured at OD₆₀₀ after infection with λ*^{cas12f1}galK*-N₂₀ and λ*^{cas12f1}galK*-N₁₆ phages. Gray arrows indicate the time point of phage infection. Each OD₆₀₀ measurement value represents an average value obtained from three independent cultures. Downward gray arrows indicate the time point of phage infection, and upward red arrows indicate the time point of sample collection for PCR. Each OD₆₀₀ measurement value represents an average value obtained from three independent cultures.

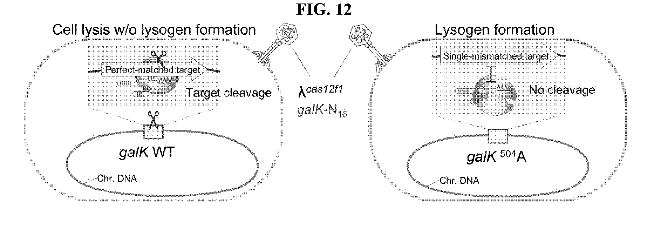

FIG. 12 schematically illustrates the mechanism of recognition of a single nucleotide variation and suppression of lysogenic strain generation by λ*^{cas12f1}galK*-N₁₆ phage. The λ*^{cas12f1}galK*-N₁₆ phage carries truncated *sgRNA* targeting the *galK* WT gene, and the Cas12f1-truncated sgRNA complex delivered by the phage cleaves the *galK* WT target through perfect base pairing to prevent lysogenization, whereas in *galK* ⁵⁰⁴A cells, a lysogenic cell may be formed because the genomic target is not recognized due to the presence of a single mismatch.

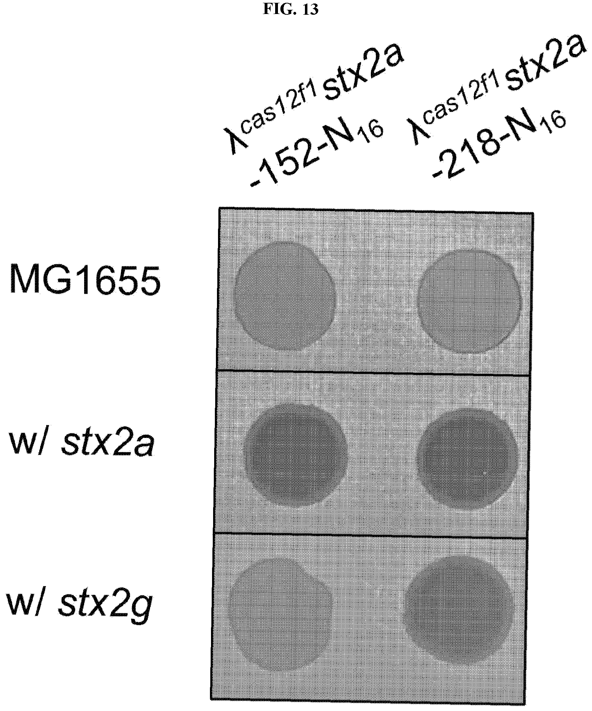

FIG. 13 shows spotting assay results of synthetic λ*^{cas12f1}stx2a*-152-N₁₆ and λ*^{cas12f1}stx2a-*218-N₁₆ phages on soft agar containing various *E*. *coli* cells having *stx2* subtypes. The *stx2a* and *stx2g* genes were inserted into the *srl* operon of the MG1655 genome.

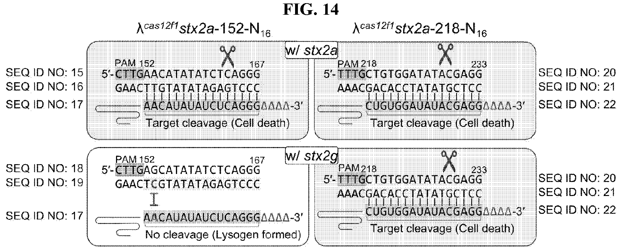

FIG. 14 shows target nucleotide sequences of *stx2* gene subtypes recognized by truncated sgRNA (N₁₆) of the CRISPR-Cas12f1 system. The nucleotides indicated in red bold type represent single nucleotide variations specific to the *stx2* subtype, which do not form perfect base pairing with the target recognition sequence of the sgRNA.

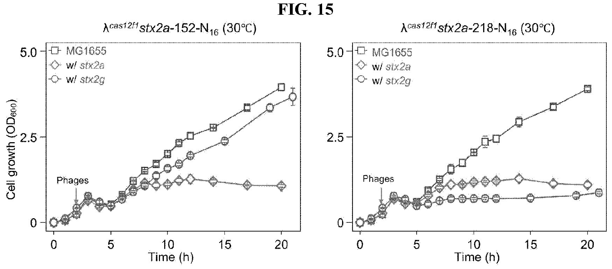

FIG. 15 shows results indicating genome *stx2* subtype sequence-specific bacterial control using a *cas12f1-sgRNA*-loaded phage. The growth of MG1655 strains having *stx2a* and *stx2g* genes was monitored at 30 °C after infection with synthetic λ*^{cas12f1}stx2a*-152-N₁₆ and λ*^{cas12f1}stx2a*-218-N₁₆ phages. Gray arrows indicate the time point of phage infection. Each OD₆₀₀ measurement value represents an average value obtained from three independent cultures.

### [Best Mode]

Terms used in the present specification are used only for the purpose of description and should not be construed as limiting.

Singular expressions include plural expressions unless the context clearly indicates otherwise. As used herein, terms such as "comprise" or "have" are intended to designate the presence of features, numbers, steps, operations, elements, parts, or combinations thereof described in the specification, and should not be understood as precluding in advance the presence or possibility of addition of one or more other features, numbers, steps, operations, elements, parts, or combinations thereof.

Additionally, unless defined otherwise, all terms used herein, including technical or scientific terms, have the same meanings as commonly understood by one of ordinary skill in the art to which the example belongs. Terms such as those defined in commonly used dictionaries should be construed as having meanings consistent with their meanings in the context of the related art, and are not to be construed in an idealized or overly formal sense unless expressly defined in the present application.

Hereinafter, the present invention will be described in detail.

The present invention provides a method of killing a microbial strain, comprising treating a microbial strain with a bacteriophage having inserted into the genome a CRISPR-Cas (Clustered Regularly Interspaced Short Palindromic Repeats/CRISPR-associated nuclease)system comprising a donor nucleic acid molecule and a gRNA (guide RNA) that complementarily bind to a target DNA, which is a gene specific to the microbial strain.

As used herein, the term "target DNA" refers to DNA that is a target for editing by the CRISPR/Cas system. In the present invention, the target DNA refers to a region of the genome of a microbial strain that is completely complementary or substantially complementary to sgRNA or crRNA. In some embodiments, the target DNA is located completely adjacent to a PAM sequence in the genome of an organism (a PAM sequence located completely adjacent to the 5' end of the target region), and may have a length of 10 nt to 40 nt.

In the present invention, the target DNA may be specific to a target microbial strain, may be shared among different strains, may be present in most bacteria, and may be present in an antibiotic resistance gene, a toxin gene, or a pathogenicity island.

As used herein, the term "PAM (protospacer-adjacent motif)" refers to a short sequence essentially required for a Cas protein to recognize and cleave target DNA in the CRISPR-Cas system. The PAM sequence serves to help the Cas protein accurately recognize and cleave DNA, and is generally composed of a specific nucleotide sequence.

As used herein, the term "donor nucleic acid molecule" or "donor nucleic acid sequence" refers to a natural or modified polynucleotide, an RNA-DNA chimera, a DNA fragment, a PCR-amplified ssDNA fragment, a PCR-amplified dsDNA fragment, or an analogue thereof, including a nucleotide sequence intended to be inserted into target DNA. Such donor nucleic acid molecules may include any form, for example, a single-stranded form and a double-stranded form, so long as they may induce modification on the target DNA and achieve the object of the present invention.

As used herein, the term "gRNA (guide RNA)" refers to an RNA molecule that binds to a Cas protein in the CRISPR-Cas system to guide the Cas protein to a target DNA sequence and enables genome editing by accurately recognizing and cleaving the target DNA sequence. The gRNA may be dual RNA including crRNA (CRISPR RNA) and tracrRNA (transactivating crRNA) that hybridize with target DNA, or may be a single-stranded guide RNA (sgRNA) including portions of the crRNA and tracrRNA and hybridizing with target DNA, and in one example of the present invention, the gRNA is sgRNA. Any gRNA may be used in the present invention so long as the gRNA includes essential portions of crRNA and tracrRNA and a portion complementary to the target.

The gRNA may be delivered to a cell or organism in the form of RNA or in the form of DNA encoding the gRNA. Additionally, the gRNA may be in the form of isolated RNA, RNA included in a viral vector, or a form encoded in a vector. Preferably, the vector may be a viral vector, a plasmid vector, or an *Agrobacterium* vector, but is not limited thereto.

As used herein, the term "Cas protein" refers to an essential protein component in the CRISPR/Cas system, and when forming a complex with two RNAs called crRNA and transactivating crRNA, forms an active endonuclease or nickase. Information on Cas genes and proteins may be obtained from GenBank of the National Center for Biotechnology Information (NCBI), but is not limited thereto. The Cas proteins are divided into two classes depending on whether there are multiple types of Cas proteins (class 1) or a single type of Cas protein (class 2), and the two classes are further subdivided into six types (I-VI) according to CRISPR-Cas loci on the genome and the type of Cas protein acting therein. Any Cas protein may be used so long as it may achieve the object of the present invention, but is preferably Cas12f1 or Cas12a. Any nucleic acid encoding the Cas protein or any Cas protein may be used so long as it may achieve the object of the present invention, but may be Cas12f1 derived from *Acidibacillus sulfuroxidans* or Cas12a.

In one example of the present invention, a lysogenic strain was obtained by treating an *E*. *coli* MG1655 strain with bacteriophage λ having the *ea59, ea31*, and *ea47* genes of the b2 region removed and the *cas12f1* gene inserted. sgRNA targeting *galK* of *E*. *coli* is expressed from a plasmid or is loaded into and expressed from the phage genome to form a complex with Cas12f1, and cleaves target DNA to suppress formation of lysogenic strains and kill microorganisms.

In the present invention, the gRNA may have a portion of nucleotides at the 3'end truncated. So long as the object of the present invention may be achieved, the number of truncated nucleotides is not limited, but preferably, the number of truncated nucleotides may be 1 to 10 (1, 2, 3, 4, 5, 6, 7, 8, 9, or 10).

In one example of the present invention, a guide RNA having four nucleotides truncated from the 3' end (3'-truncated) of the guide RNA including a nucleotide sequence complementary to target DNA includes a region consisting of 16 consecutive nucleotides complementary to the target DNA, and may identify a single nucleotide variation in the target DNA, thereby overcoming mismatch tolerance and enabling precise gene editing.

In the present invention, the method of the present invention does not limit the type of bacteriophage so long as the intended effect may be achieved, and for example, bacteriophages such as P1, P2, λ, and T-series phages from T1 to T7 may be used in the method.

In the present invention, the bacteriophage may have both a lytic life cycle and a lysogenic life cycle.

In the present invention, the method of the present invention does not limit the type of microorganism so long as the intended effect may be achieved, and for example, bacteria such as *Escherichia coli, Bacillus subtilis, Lactobacillus rhamnosus, Salmonella enterica, Streptococcus thermophilus, Listeria, Campylobacter*, or *Staphylococcus aureus* may be used in the method.

Additionally, the present invention provides a method of constructing a bacteriophage, comprising inserting into a bacteriophage genome a CRISPR-Cas system comprising a donor nucleic acid molecule and gRNA that complementarily bind to a target DNA, which is a gene specific to the microbial strain.

As used herein, the terms "inserting," "introducing," "delivering," and "administering" (and grammatical variations thereof) mean presenting a polynucleotide of interest to a host organism or a cell of the organism (for example, a host cell, such as a bacterial cell) in a manner such that the polynucleotide gains access to the interior of the cell, and such methods include "transformation," "transfection," and/or "transduction."

Additionally, a lytic bacteriophage having a genome edited and converted into a lytic life cycle pathway according to the above-described method of the present invention is provided, and the lytic bacteriophage may prevent formation of a lysogenic microbial strain and may avoid superinfection immunity.

Additionally, the present invention provides a method of avoiding superinfection immunity of a lysogenic microbial strain, comprising treating a host microbial strain with the bacteriophage.

Additionally, the present invention provides a composition for avoiding superinfection immunity of a lysogenic microbial strain, comprising the bacteriophage.

In the present invention, duplicated contents or terms are omitted in order to avoid excessive complexity of the present specification.

That is, bacteriophages to which the gene editing technology of the present invention is applied may contribute to solving problems of existing antibiotics (emergence and spread of antibiotic resistance). For example, as a means for treating increasing antibiotic-resistant bacterial infections, it is expected that various resistant bacteria that neutralize existing antibiotics may be killed and infectious diseases caused by resistant bacteria may be treated using Phage Therapy.

Hereinafter, the examples are provided only for more specifically describing the present invention, and it will be obvious to one of ordinary skill in the art to which the present invention belongs that the scope of the present invention is not limited by these examples according to the gist of the present invention.

### <Example> Experimental method

### Strains and culture

Bacterial strains used in the present invention are shown in Table 1. These strains were cultured in LB medium at 30 or 37 °C according to the specific requirements of each strain. To produce competent cells, the *E*. *coli* strains were cultured overnight at 30 °C and then inoculated into LB medium at a final concentration of 1%. The cell culture was maintained at 30 °C until the OD₆₀₀ reached 0.4, at which time harvesting was performed. MG1655 and HL051 strains harboring the pKD46 plasmid (pSC101ori^{ts} *araC* λ *red* genes AmpR) along with λ red recombinase were further cultured with L-arabinose at the final concentration of 1 mM for 3 hours. The cell culture was then washed twice with 10% glycerol and aliquoted at a volume of 50 µL for storage at -80 °C. Depending on the selection marker for the plasmid or gene cassette, ampicillin, kanamycin, chloramphenicol, and spectinomycin were respectively added to the medium at final concentrations of 50, 25, 12.5, and 75 µg/mL.

**[Table 1]**

| Strain | Characteristics |
|---|---|
| MG1655 | *F- ilvG rfb-50 rph-1* |
| ATCC 43895 | *E coli* O157:H7, clinical isolate, *stx1+ stx2+* |
| HK1020 | BL21(DE3), *kgtp*-GGGGS linker-*sfgfp-CmR* |
| HL002 | MG1655 Δ*araBAD* carrying *P*_{BAD}-*cas9*-KmR *galK* ⁵⁰⁴AT |
| HL051 | MG1655, λ *cl*⁸⁵⁷ lysogen |
| HL059 | MG1655 *galK* ⁵⁰⁴AT |
| HL061 | MG1655, *araBAD*::*P*_{BAD}*-Ascas12f1-*Km^{R} |
| HL062 | MG1655, λ *cl*⁸⁵⁷ Δb2 lysogen carrying *Ascas12f1*-CmR-FRT |
| HL066 | MG1655 *ΔgalK* |
| HL080 | MG1655 *ΔgalKΔxylB,* λ *c*/⁸⁵⁷ Δb2 tysogen carrying *Ascas12f1*-CmR-FRT |
| HL081 | MG1655, λ *cl*⁸⁵⁷ Δb2 lysogen carrying *Ascas12f1* plus *sgRNA*-KmR-FRT (Target: ⁴⁹⁷TAGGCTGTAACTGCGGGATC⁵¹⁶ in *galK*) |
| HL085 | MG1655 Δ*srlAEBD* carrying *stx2a*-CmR-FRT |
| HL086 | MG1655 Δ*srlAEBD* carrying *stx2a* A¹⁵³G-CmR-FRT |

### Construction of lysogenic strains

150 µL of the MG1655 strain cultured overnight was added to 15 mL of 0.6% soft agar supplemented with CaCl₂ (5 mM) and MgSO₄ (10 mM), mixed, and then inoculated onto an LB agar plate (diameter: 90 mm). After air-drying for 20 minutes, 4 µL of λ *cI⁸⁵⁷* phage lysate was spotted on the agar and incubated at 21 °C for one week. Colonies from faint spots were isolated, and the formation of the lysogenic strain, HL051 (λ-lysogenic MG1655) was confirmed through PCR using the attB_F + λint_R primer pair that targets the bacterial and phage genomes. The primers used in the present invention are shown in Table 2 below.

**[Table 2]**

| Primer | Sequence (5'→3') |
|---|---|
| sgRNA_sacl_2F_N | GCCTACAATCCCCGGTATACGAATTCCGAGCTCAAAAAAAAGGCGGTGATGTAAACAC (SEQ ID NO: 1) |
| sgRNA_KmR_N2_F | GCAAAAGGGGATGATAAGTTTGGGATCCGTATACCGTGTAGGCTGGATTCCGGGGATCC (SEQ ID NO: 2) |
| sgRNA_sacl_2R_N | ACCGCCTTTTTTTTGAGCTCGGAATTCGTATACCGGGGATTGTAGGCTGGAGCTGCTTC (SEQ ID NO: 3) |
| KmR_sgRNA_N2_R | CCCGGAATCCAGCCTACACGGTATACGGATCCCAAACTTATCATCCCCTTTTGCTTATG (SEQ ID NO: 4) |
| attB_F | ATCGGGGAAGGATTCCACGCTGCAGC (SEQ ID NO: 5) |
| bioB_R | GACAAGCTCCGGTCTTAATCGACAGC (SEQ ID NO: 6) |
| λint_R | ACTCGTCGCGAACCGCT (SEQ ID NO: 7) |

### Host engineering

In *E. coli* HL059 (MG1655 *galK* ⁵⁰⁴AT), the *galK* gene was edited using CRISPR-Cas9, the *cas9* gene of the arabinose operon was deleted through P1 transformation, and then the original *araBAD* operon was reintroduced. *E. coli* HL066 (MG1655 Δ*galK*) was constructed by introducing the Δ*galK* variant of the Keio collection JW0740 strain into MG1655 through P1 transformation. To produce an MG1655 strain harboring the *stx2* gene, the *stx2a* gene was amplified using genomic DNA from ATCC 43895 as a template, a *stx2a*-CmR cassette was constructed by replacing the *srlAEBD* operon in the MG1655 genome and then inserted. The *stx2a-CmR* cassette was inserted into L-arabinose-induced MG1655 cells harboring the pKD46 plasmid through electroporation using a 0.1 cm cuvette at 25 µF, 200 Ω and 1.8kV. All subsequent electroporations were performed under the same conditions. After electroporation, 950 µL of SOC medium was added to the cells, which were then recovered at 30 °C for 1 hour, plated onto chloramphenicol-containing LB agar, and then cultured at 37 °C. The grown colonies were confirmed by PCR, and further verified by Sanger sequencing to confirm whether the resulting HL085 strain had the *stx2a*-CmR cassette. Nucleotide sequences for *stx2* subtypes were referenced from the following NCBI accession numbers: *stx2a* (CP008957) and *stx2g* (AY286000). The genomic DNA of the HL085 strain was used as a template for overlap PCR to amplify the *stx2g-CmR* cassette, and a HL086 *(MG1655-stx2g)* strain was produced in the same manner as the HL085 (MG1655-*stx2a*) strain.

### Integration of cas12f1

To insert the *cas12f1* gene into the b2 region of the λ phage genome from which *ea59*, *ea47*, and *ea31* genes were deleted, a *cas12f1*-CmR cassette was constructed. The *PrpsL*-*cas12f1* fragment was activated using p15a-AsCas12f-apmR (obtained from Quanjiang Ji, Addgene plasmid #171610) as a template. The CmR cassette was amplified using HK1020 genomic DNA as a template. After generating the *PrpsL-cas12f1*-CmR cassette through overlap PCR, it was electroporated into L-arabinose-induced HL051 (λ *cI⁸⁵⁷* lysogenic strain) cells harboring the pKD46 plasmid. The electroporated cells were plated onto LB agar plates containing chloramphenicol and cultured at 30 °C. The resulting colonies were initially confirmed by PCR and subsequently verified by Sanger sequencing to confirm whether the *cas12f1*-CmR cassette was inserted into the b2 region of the λ prophage genome in the finally-constructed HL062 strain (a λ *cI⁸⁵⁷* lysogenic strain carrying the *cas12f1* gene).

### Integration of cas12f1-sgRNA

Using the pHL267 plasmid as a template, an sgRNA gene was amplified with the primer pair sgRNA_sacI_2F_N + KmR_sgRNA_N2_R. The 667 bp fragment of the *cas12f1* gene was amplified using genomic DNA from the HL061 strain as a template. The KmR cassette was amplified using the genomic DNA of the HL002 strain and the primer pair sgRNA_KmR_N2_F + sgRNA_sacI_2R_N. After constructing a *cas12f1* (667 bp)-*sgRNA*-KmR cassette through overlapping PCR, which was electroporated into a HL080 strain (a λ *cI⁸⁵⁷* lysogenic strain harboring the *cas12f1* gene and Δ*galK* Δ*xylB* mutation). The electroporated cells were plated onto kanamycin-containing LB agar plates and cultured at 30 °C. Colony PCR and Sanger sequencing were performed to confirm the construction of a HL081 strain (a λ *cI⁸⁵⁷* lysogenic strain carrying the *cas12f1* gene and *sgRNA* targeting *galK*). Subsequently, a synthetic phage with a modified *sgRNA* target recognition sequence (TRS) within the prophage genome was constructed using the HL081 genome as a template through overlapping PCR.

### Spotting assay

Various phages lysogenic cells were cultured overnight at 30 °C. The supernatant of this cell culture was spotted on soft agar plates to produce phage plaques. The cell culture obtained by culturing MG1655 overnight was inoculated into LB medium supplemented with CaCl₂ (5 mM) and MgSO₄ (10 mM), and cultured at 30 °C while shaking at 180 rpm until the OD₆₀₀ reached 0.4. Afterward, phage plaques were added to this bacterial culture, and the mixture was incubated at 42 °C until complete lysis. A supernatant containing the phage lysate obtained through centrifugation (3,000 rpm, 4 °C, 30 minutes) was used to quantify plaque-forming units (pfu), which was then stored at 4 °C after adding 0.1% chloroform. A spotting assay was performed to confirm the infectivity of the engineered phages and their ability to form clear or turbid spots. Specifically, 150 µL of the overnight cell culture of each host strain was added to 15 mL of 0.6% soft agar supplemented with CaCl₂ (5 mM) and MgSO₄ (10 mM), mixed, and then plated onto LB agar plates (diameter: 90 mm). Next, 4 µL of the phage lysate was spotted on the soft agar plate. The plates were incubated at 30 °C for 24 hours and at 37 °C for 16 hours, respectively, according to the specific requirements of each strain.

### Broth culture

To measure culture growth, a single colony of each host strain was cultured overnight as a starter culture in LB medium at 30 or 37 °C while shaking at 180 rpm. For this culture, the overnight culture was inoculated at a final concentration of 1% into a 1 L flask containing 200 mL of LB medium, and cultured at 180 rpm at 30 or 37 °C until OD₆₀₀ reached 0.4. Subsequently, 49 mL of the culture was transferred to each 125 mL disposable flask. To achieve a multiplicity of infection (MOI) of 0.1, 1 mL of phage lysate was diluted and added to each flask, and then incubated at 30 or 37 °C. OD₆₀₀ measurement was carried out using a spectrophotometer. Nineteen hours after phage addition, each culture was plated on LB agar plates, incubated at 30 °C for 18 hours, and then streaked on kanamycin-containing LB plates to obtain single colonies. The presence of the lysogenic strain in the obtained single colony was confirmed by PCR using primer pairs attB_F + λint_R and attB_F + bioB_R.

### <Experimental Example 1> Lysogeny of bacteriophage λ in E. coli

The turbidity of spots formed on soft agar plates inoculated with host MG1655 and various phages, λ *cI⁸⁵⁷,* λ Δ*b2* (nonessential gene deletion), and synthetic lytic phage *λ cI^{antisense}*, was compared.

As a result, as shown in FIG. 1, it was confirmed that λ *cI⁸⁵⁷* formed a turbid spot at 30 °C and a clear spot at 37 °C. This indicates that normal lysogeny occurs at 30 °C, but at 37 °C, a heat-sensitive CI⁸⁵⁷inhibitor is inactivated, initiating the λ lysis cycle. In contrast, the synthetic lytic phage λ *cI^{antisense}* formed clear spots at all temperatures as expected. In addition, λ Δ*b2* formed a turbid spot at 30 °C and a clear spot at 37 °C, similar to λ *cI⁸⁵⁷*. These results suggest that the deletion of the b2 region does not affect the phage life cycle.

Subsequently, after inoculating the liquid culture of host MG1655 with λ *cI⁸⁵⁷,* λ Δ*b2*, and λ *cI^{antisense}* phages, its growth was measured and compared with the results obtained on solid media.

As a result, as shown in FIG. 2, when MG1655 was infected with λ *cI^{antisense}* at 30 °C, complete lysis occurred after 3 hours. Growth resumed at the 12-hour point on the graph, reaching an OD₆₀₀ value of 3.0 after 19 hours. The cells were lysed by infection with λ *cI⁸⁵⁷* and λ *Δb2*, but regrew 4 hours after infection, reaching an OD₆₀₀ value of 4.2 at 19 hours. The cells that had grown again at 19 hours (shown on the graph) were no longer infected with λ *cI*⁸⁵⁷, and PCR results confirmed that cells surviving the λ *cI^{antisense}* infection were not lysogenic. However, the cells infected with λ *cI⁸⁵⁷* and λ Δ*b2* were confirmed to be lysogenic.

Furthermore, as shown in FIG. 3, when MG1655 was cultured at 37°C and separately infected with λ *cI⁸⁵⁷,* λ Δ*b2,* and λ *cI^{antisense}*, the host regrowth patterns after lysis were identical. None of the cells regrown at 37°C were infected with λ *cI⁸⁵⁷*, and PCR results confirmed that they were not lysogenic.

These results demonstrate that λ *cI⁸⁵⁷* is lysogenic at 30 °C under both solid and liquid culture conditions, but not at 37 °C. In addition, it can be seen that removal of the non-essential b2 region from λ Δ*b2* does not significantly affect the phage's ability to infect hosts and form lysogens at 30 °C.

### <Experimental Example 2> Complete lysis of E. coli by bacteriophage λ using Cas12f1 nuclease

λ*^{cas12f1}* phages were constructed by inserting the *cas12f1* gene into the position where the b2 region was removed from a genome (FIG. 4). To test the function of the phages, E. coli cells harboring various *galK* target sequences, *galK* ⁵⁰⁴A, *galK* ⁵⁰⁴AT, and Δ*galK*, were engineered, and the turbidity of spots formed by the λ*^{cas12f1}* phage on agar at 30 °C was observed based on the presence or absence of *galK* target sgRNA plasmids.

As a result, as shown in FIG. 5, in the sgRNA plasmid-harboring cells, when the λ*^{cas12f1}* phage was spotted on the *galK* WT and *galK* ⁵⁰⁴A cells, clear spots were formed, whereas in the *galK* ⁵⁰⁴AT and Δ*galK* cells, turbid spots were formed. In all cells lacking the sgRNA plasmid, turbid spots were formed.

These results indicate that the expressed Cas12f1 and sgRNA derived from a host sgRNA plasmid form a Cas12f1-sgRNA complex that effectively recognizes and cleaves *galK* WT and *galK* ⁵⁰⁴A targets, inducing cell death and clear spot formation (FIG. 6A). However, in the presence of two nucleotide (nt) mismatches (*galK* ⁵⁰⁴AT) or in the absence of the *galK* target (Δ*galK*), the Cas12f1-sgRNA complex fails to cleave the genome, preventing cell death and forming turbid spots due to the formation of lysogens (FIG. 6B).

Next, to distinguish *galK* WT and *galK* ⁵⁰⁴A, which differ by one nucleotide, from each other, the effect of a 3'-truncated sgRNA approach for overcoming the mismatch tolerance of the Cas12f1 system was tested.

The same λ*^{cas12f1}* phage spotting assay was performed using sgRNAs with the length of target recognition sequence (TRS) ranging from 20 nt (Δ0) to 15 nt (Δ5).

As shown in FIG. 7A, clear spots were formed when the *galK* WT cells harboring Δ0 to Δ4 nt sgRNA plasmids were infected with the λ*^{cas12f1}* phages. However, in the *galK* ⁵⁰⁴A cells, clear spots were formed only with Δ0 to Δ3 nt sgRNA plasmids, whereas turbid spots were formed in the cells harboring the Δ4 nt sgRNA plasmid.

In addition, when phage spotting assays were performed at 37 °C, clear spots were formed on all plates regardless of the strain or phage type (data not shown).

To confirm whether various Cas systems, other than Cas12f1, can be integrated into and applied to phages, the *cas12a* gene was inserted into the position where the b2 region was removed in the genome, thereby constructing λ*^{cas12a}* phages. To test the function of the phages, the same λ*^{cas12a}* phage spotting assay was performed using crRNAs with the length of target recognition sequence (TRS) ranging from 23 nt (Δ0) to 15 nt (Δ8) by the same method as described above.

As a result, as shown in FIG. 7B, when *galK* WT cells harboring Δ0 to Δ7 nt crRNA plasmids was infected with the λ*^{cas12f1}* phage, clear spots were formed. However, in the *galK* ⁵⁰⁴A cells, clear spots were formed only with the Δ0 to Δ6 nt crRNA plasmids, whereas turbid spots were formed in the cells harboring the Δ7 nt crRNA plasmid.

These results demonstrate that when host cells were infected with bacteriophages harboring Cas12f1 or Cas12a, truncated sgRNA or crRNA can distinguish single nucleotide mutations in the target gene (FIG. 8) and control host cell lysogen formation and cell death.

### <Experimental Example 3> Genomic DNA sequence-specific lysis of E. coli through λ phage-mediated delivery of Cas12f1 or Cas12a and truncated sgRNA or crRNA

Synthetic phage λ^{*cas12f1*-*sgRNA*} or λ^{*cas12a*-*crRNA*} was designed by integrating *cas12f1* or *cas12a*, and a *galK*-targeting sgRNA or crRNA gene into the b2 region of the bacteriophage λ genome, and experiments were then conducted to confirm its ability to distinguish single nucleotide mutations in the target sequence of the host genome and its effect on lysogeny and cell lysis.

λ*^{cas12f1}galK*-N₂₀ and λ*^{cas12f1}galK*-N₁₆ respectively having a TRS length of 20 nt (Δ0) and 16 nt (Δ4), or λ*^{cas12a}galK*-N₂₃ and λ*^{cas12a}galK*-N₁₆ respectively having a TRS length of 23 nt (Δ0) and 16 nt (Δ7) were constructed (FIG. 9). PCR results confirmed that the *cas12f1* or *cas12a*, and the *sgRNA* or *crRNA* gene were inserted into λ *cI⁸⁵⁷* genome, and the base sequences of the sgRNA and crRNA genes were identified through Sanger sequencing.

Spotting assays were performed by spotting *E*. *coli* MG1655 cells with various *galK* genotypes (*galK* WT, *galK* ⁵⁰⁴A, *galK* ⁵⁰⁴AT, and Δ *galK*) with engineered phages λ*^{cas12f1}galK-*N₂₀, λ*^{cas12f1}galK*-N₁₆ and λ *cI^{antisense}*, or λ*^{cas12a}galK*-N_{23,} λ*^{cas12a}galK*-N₁₆ and λ *cI^{antisense}* on soft agar plates, and incubating the cells at 30 °C for 16 hours.

When spotting λ*^{cas12f1}galK*-*sgRNA*-carrying phages, as shown in FIG. 10A, it was confirmed that clear spots were formed by the λ*^{cas12f1}galK*-N₂₀, λ*^{cas12f1}galK*-N₁₆ or λ*^{cas12a}galK-*N₂₃- λ*^{cas12a}galK*-N₁₆, and λ *cI^{antisense}* phages on the soft agar of the *galK* WT cells. On the soft agar of galK ⁵⁰⁴A cells, only the λ^{cas12f1}galK-N₂₀ phage and the λ *cI^{antisense}* lytic phage formed clear spots, whereas the λ*^{cas12f1}galK*-N₁₆ phage formed a turbid spot.

When spotting λ*^{cas12a}galK*-*crRNA*-carrying phages, as shown in FIG. 10B, it was confirmed that the λ*^{cas12a}galK-*N₂₃, λ*^{cas12a}galK*-N₁₆, or λ *cI^{antisense}* phages formed clear spots on the soft agar of *galK* WT cells. On the soft agar of *galK* ⁵⁰⁴A cells, only the λ*^{cas12a}galK*-N₂₃ phage and the λ *cI^{antisense}* lytic phage formed clear spots, whereas the λ*^{cas12a}galK*-N₁₆ phage formed a turbid spot.

In Δ*galK* host cells, only the λ *cI^{antisense}* lytic phage formed a clear spot, whereas the other phages formed turbid spots. In the *galK* ⁵⁰⁴AT host cells, λ*^{cas12f1}galK*-N₂₀, λ*^{cas12f1}galK*-N₁₆, and λ*^{cas12a}galK*-N_{≥16} formed turbid spots, whereas λ*^{cas12a}galK*-N₂₃ formed a clear spot (FIG. 10B). As with other host cells, the λ *cI^{antisense}* lytic phage showed a clear spot.

The above results showed that, when host cells were infected with λ*^{cas12f1}galK*-N₁₆ or λ*^{cas12a}galK*-N₁₆ phages, the loaded Cas12f1-cleaved sgRNA (*galK*-N₁₆) complex or Cas12a-cleaved crRNA (*galK*-N₁₆) complex recognizes and cleaves the *galK* WT target, preventing lysogen formation, whereas it fails to differentially recognize and cleave the *galK* ⁵⁰⁴A target.

All phages formed clear spots in all hosts when cultured at 37 °C after spotting (data not shown).

Next, *galK* WT, *galK* ⁵⁰⁴A, ⁵⁰⁴AT, and Δ*galK* host cells were infected with λ *cI⁸⁵⁷*, λ*^{cas12f1}galK*-N₂₀, λ*^{cas12f1}galK*-N₁₆, and λ *cI^{antisense}* phages, and their growth was monitored in liquid culture.

As a result, as shown in FIG. 11, in the *galK* WT cells infected with λ*^{cas12f1}galK*-N₂₀ and λ*^{cas12f1}galK*-N₁₆ phages at 30 °C, cell growth resumed after lysis, reaching an OD₆₀₀ value of approximately 1.0. In contrast, for *galK* ⁵⁰⁴A cells, only those infected with λ*^{cas12f1}galK*-N₂₀ phage showed low growth, whereas cells infected with the λ^{cas12f1}*galK*-N₁₆ phage reached an OD₆₀₀ value of 3.8, similar to that of cells infected with the λ *cI⁸⁵⁷* phage.

In the *galK* WT cells, streaking and PCR verification revealed that no lysogens were formed from the cell cultures that were grown following lysis induced by λ*^{cas12f1}galK*-N₂₀ and λ*^{cas12f1}galK*-N₁₆ phage infection at 30°C. However, in the *galK* ⁵⁰⁴A cells, five colonies induced from the λ*^{cas12f1}galK*-N₁₆ infection were confirmed to be lysogenic, which is consistent with the spotting assay results indicating lysogen formation by λ*^{cas12f1}galK*-N₁₆ in *galK* ⁵⁰⁴A cells. As confirmed by colony PCR, the infection of *galK* ⁵⁰⁴A cells with λ*^{cas12f1}galK*-N₂₀ did not result in lysogen formation. The liquid cultures of *galK* ⁵⁰⁴AT and Δ*galK* cells infected with λ*^{cas12f1}galK-*N₂₀ and λ*^{cas12f1}galK*-N₁₆ phages showed growth curves nearly identical to that infected with the λ *cI⁸⁵⁷* phage (data not shown). This indicates that the Cas12f1-sgRNA complex delivered by the phages did not recognize any targets in the ⁵⁰⁴AT and Δ*galK* cells.

When monitoring the growth of *galK* WT cells and the *galK* ⁵⁰⁴A strain at 37 °C, the λ*^{cas12f1}galK*-N₂₀ and λ*^{cas12f1}galK*-N₁₆ phages showed growth patterns nearly identical to that of the λ *cI⁸⁵⁷* phage lacking Cas12f1-sgRNA (data not shown). This similarity arises because the inactivation of heat-sensitive CI⁸⁵⁷ results in the phages to exclusively undergo the lytic cycle, and the presence of Cas12f1-sgRNA becomes irrelevant.

These results demonstrate that synthetic phages carrying both the *cas12f1* and *sgRNA* genes can effectively recognize and cleave the target *galK* gene in host cells, thereby suppressing lysogen formation. Furthermore, the phages carrying the truncated sgRNA gene can identify single nucleotide mutations in target DNA (FIG. 12).

### <Experimental Example 4> Precise control of E. coli harboring toxin gene

Some pathogenic microorganisms such as *Shigella* and *E. coli* express Shiga toxin, which may be classified into type 1 and type 2 variants, with the subtype *stx2a* known to be the most toxic.

*E*. *coli* strain MG1655 harboring *stx2a* and *stx2g* genes in the *srlAEBD* operon was engineered. Phages λ*^{cas12f1}stx2a-*152-N₁₆ and λ*^{cas12f1}stx2a*-218-N₁₆ that target nucleotides 152-167 (16 nt) and 218-233 (16 nt) of the *stx2a* gene, respectively, were constructed (data not shown). Next, the efficacy of the phages carrying a truncated sgRNA and a Cas12f1 system targeting the *stx2a* gene was investigated for inducing lysis and preventing lysogeny in *E*. *coli* MG1655 strains harboring different *stx2* gene subtypes.

According to the spotting assay results, as shown in FIG. 13, the λ*^{cas12f1}stx2a*-152-N₁₆ phage formed a clear spot only in the strain harboring the *stx2a* gene, whereas the λ*^{cas12f1}stx2a-*218-N₁₆ phage formed clear spots in both the *stx2a* and *stx2g* gene-harboring strains. This indicates that a lysogenic host was formed as seen from the turbid spots, because the λ*^{cas12f1}stx2a-*152-N₁₆ phage failed to recognize the *stx2g* gene target due to a single nucleotide mismatch (FIG. 14).

Consistent with the spotting assay results in flask culture, the λ*^{cas12f1}stx2a*-152-N₁₆ phage suppresses the growth of the strain harboring the *stx2a* gene, whereas the strain harboring the *stx2g* gene showed a growth curve similar to that of the strain infected with the λ *cI⁸⁵⁷* phage (FIG. 15). However, the λ*^{cas12f1}stx2a*-218-N₁₆ phage suppressed the growth of both the *stx2a* and *stx2g* gene-carrying strains due to perfect base pairing between the target DNA and the truncated sgRNA.

In addition, the same growth pattern was observed in all four types of strains when no phage was introduced or when the cells were infected with the λ *cI⁸⁵⁷* phage (data not shown). These results demonstrate that synthetic λ phages carrying *cas12f1* and truncated *sgRNA* can distinguish single nucleotide mutations, enabling specific control of subtypes of the *stx2* virulence gene in the host genome.

While specific parts of the present invention have been described in detail above, it is clear to those skilled in the art that these specific parts are merely preferred embodiments, and the scope of the present application is not limited thereto. Thus, the substantial scope of the present invention will be defined by the accompanying claims and their equivalents.

## Claims

1. A method of killing a microbial strain, which comprises treating a microbial strain with a bacteriophage,
wherein the bacteriophage comprises a CRISPR-Cas (Clustered Regularly Interspaced Short Palindromic Repeats/CRISPR-associated nuclease) system inserted into the genome, the CRISPR-Cas system comprising a donor nucleic acid molecule and a guide RNA (gRNA), wherein the donor nucleic acid molecule and the guide RNA (gRNA) complementarily bind to a target DNA, which is a gene specific to the microbial strain.

2. The method of claim 1, wherein the Cas is Cas12f1 or Cas12a.

3. The method of claim 1, wherein the gRNA has a truncated 3' end.

4. The method of claim 1, wherein the method suppresses formation of a lysogenic strain.

5. The method of claim 1, wherein the microbial strain is resistant to an antibiotic.

6. The method of claim 1, wherein the microbial strain is selected from the group consisting of *Escherichia coli*, *Bacillus subtilis*, *Lactobacillus rhamnosus*, *Salmonella enterica*, *Streptococcus thermophilus*, *Listeria*, *Campylobacter*, or *Staphylococcus aureus*.

7. The method of claim 1, wherein the bacteriophage has a lytic life cycle or a lysogenic life cycle.

8. A method of constructing a lytic bacteriophage, comprising:
inserting a CRISPR-Cas (Clustered Regularly Interspaced Short Palindromic Repeats/CRISPR-associated nuclease) system, which comprises a donor nucleic acid molecule and a guide RNA (gRNA), wherein the donor nucleic acid molecule and the guide RNA (gRNA) complementarily bind to a target DNA, which is a gene specific to a microbial strain, into the bacteriophage genome.

9. The method of claim 8, wherein the lytic bacteriophage avoids superinfection immunity of a lysogenic microbial strain.

10. A lytic bacteriophage constructed by the method of constructing a lytic bacteriophage according to claim 8.

11. A method of avoiding superinfection immunity of a lysogenic microbial strain, comprising:
treating a lysogenic microbial strain with the bacteriophage of claim 10.

12. A composition for avoiding the superinfection immunity of a lysogenic microbial strain, comprising the bacteriophage of claim 10.
